# FASCICULE DE BREVET EUROPEEN

(11) **EP 1 847 251 B1**
(45) Date de publication et mention de la délivrance du brevet: **10.06.2009**
(21) Numéro de dépôt: 07105459.7
(22) Date de dépôt: 02.04.2007
(51) Int. Cl.: A61K 8/49, A61K 8/36, A61Q 5/06, A61Q 5/10, A61Q 5/02

(54) **Utilisation d'un corps gras insature pour protéger la couleur vis-à-vis du lavage de fibres kératiniques teintées artificiellement; procédés de coloration**
Verwendung von einer ungesättigten Fettkomponente zum Schutz der Farbe gegenüber dem Auswaschen von gefärbten Keratinfasern, und dessen Färbeverfahren
Use of an unsaturated fatty compound to protect color against washing of artificially dyed keratin fibres, and dye processes

(30) Priorité: 12.04.2006 FR 0651335
(43) Date de publication de la demande: 24.10.2007
(73) Titulaire: L'Oréal, 75008 Paris (FR)
(72) Inventeur: Lalleman, Boris, 75004, PARIS (FR); Giroud, Franck, 73390, Chamoux sur Gelon (FR)
(74) Mandataire: Le Blainvaux Bellegarde, Françoise

(56) Documents cités:
- FR-A1- 2 080 759
- US-A1- 2003 198 615
- US-A1- 2004 170 590
- US-A1- 2005 144 739

## Description

L'invention a pour objet l'utilisation d'au moins un corps gras insaturé particulier de formule (I) que l'on définira plus loin en détail comme agent permettant de protéger la couleur vis-à-vis du lavage des fibres kératiniques teintes artificiellement, notamment des fibres kératiniques humaines et plus particulièrement des cheveux.

Il est connu de teindre les fibres kératiniques notamment humaines et en particulier les cheveux avec des compositions tinctoriales contenant des précurseurs de colorant d'oxydation, appelés généralement bases d'oxydation. Ces bases d'oxydation sont des composés incolores ou faiblement colorés qui, associés à des produits oxydants, donnent naissance par un processus de condensation oxydative à des composés colorés. On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser poser, puis à rincer les fibres.

Les colorations qui en résultent sont des colorations particulièrement chromatiques qui sont cependant temporaires ou semi-permanentes car la nature des interactions qui lient les colorants directs à la fibre kératinique et leur désorption de la surface et/ou du coeur de la fibre sont responsables de leur faible puissance tinctoriale et de leur mauvaise tenue aux lavages.

La couleur artificielle des cheveux apportée par un traitement de coloration directe ou d'oxydation s'estompe progressivement du fait des lavages répétés et conduit dans le temps à un affadissement de la coloration des cheveux. L'utilisation des produits soins rincés et non rincés commercialisés n'améliore pas suffisamment la tenue de la couleur artificielle des cheveux.

Il est donc nécessaire de mettre au point des moyens permettant de protéger la couleur artificielle de l'effet des lavages répétés.

La Demanderesse a maintenant découvert de manière surprenante que l'utilisation de corps gras insaturés particuliers de formule (I) que l'on définira plus loin en détail permettait de protéger la couleur artificielle de fibres kératiniques.

Cette découverte est à la base de la présente invention.

On entend par « fibres kératiniques humaines » par les cheveux, les poils notamment de barbe ou moustache, les cils, les sourcils.

On entend par « fibres kératiniques teintes artificiellement » par fibres kératiniques teintes par un procédé de coloration directe ou par un procédé de coloration d'oxydation.

On entend par « lavage », une ou plusieurs applications sur les fibres kératiniques d'une composition aqueuse rincée, le plus souvent détergente telle qu'un shampooing. Cette expression inclut également les baignades en particulier en mer ou en piscine.

L'invention a donc pour objet l'utilisation d'au moins un corps gras insaturé particulier de formule (I) que l'on définira plus loin en détail comme agent permettant de protéger la couleur vis-à-vis du lavage des fibres kératiniques teintes artificiellement, notamment des fibres kératiniques humaines et plus particulièrement des cheveux.

L'invention a également pour objet un procédé pour protéger la couleur vis-à-vis du lavage des fibres kératiniques teintes artificiellement, caractérisé par le fait qu'il consiste à appliquer sur lesdites fibres au moins une composition comprenant dans un milieu cosmétiquement acceptable au moins un corps gras insaturé de formule (I) que l'on définira plus loin en détail

De plus, la protection apportée par le traitement selon l'invention est durable c'est-à-dire ne nécessitant pas de réapplications fréquentes du produit.

Un autre objet de l'invention concerne un procédé de coloration consistant à appliquer sur les fibres kératiniques en particulier les fibres kératiniques humaines et plus particulièrement les cheveux, une composition (A) colorante directe ou d'oxydation pendant un temps suffisant pour développer la couleur, et de faire suivre ou précéder cette application par l'application d'une composition (B) contenant dans un milieu cosmétiquement acceptable au moins un corps gras insaturé de formule (I) que l'on définira plus loin en détail.

Les différents objets de l'invention vont maintenant être détaillés. L'ensemble des significations et définitions des composés utilisés dans la présente invention données ci-dessous sont valables pour l'ensemble des objets de l'invention.

Les corps gras insaturés conformes à l'invention répondent à la formule générale (I) suivante : 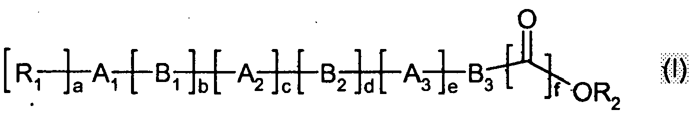 où :
A₁, A₂, A₃ , identiques ou différents, désignent un radical hydrocarboné monovalent ou divalent, linéaire ou cyclique comportant au moins une insaturation ;
B₁, B₂, B₃, identiques ou différents, désignent un radical CₙH₂ₙ où n est un entier inférieur à 20 ;
R₁ désigne un alkyle linéaire ou ramifié en C₁-C₁₂ ;
R₂ désigne hydrogène, un métal alcalin M ou le groupe sorbitane de formule : 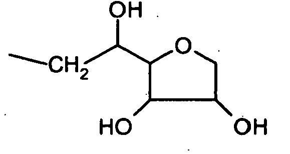
a,b,c, d, e, f , identiques ou différents, valent 0 ou 1

On entend par « insaturation » une double ou triple liaison.

Les insaturations de radical divalent étant en conformation cis ou trans, de préférence en cis.

M désigne de préférence Na+ou K+.

Les composés de formule (I) préférentiels sont choisis de préférence parmi les composés de formule (II) suivante : 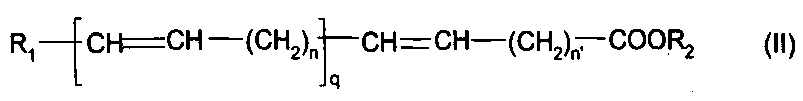 dans laquelle :
R₁ désigne un alkyle linéaire ou ramifié en C₁-C₁₂ ;
R₂ désigne hydrogène, un métal alcalin ou le groupe sorbitane de formule : 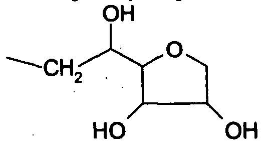
n et n', identiques ou différents, sont des entiers allant de 1 à 10 et de préférence de 1 à 8;
q vaut 0, 1 ou 2.

Parmi les composés de formule (II) préférés, on peut citer :
l'acide lauroléique;
l'acide myristoléique ;
l'acide palmitoléique
l'acide oléique ;
l'acide linoléique ;
l'acide linolénique,
l'oléate de sorbitan
et plus particulièrement l'acide oléique et l'oléate de sorbitan.

Le milieu cosmétiquement acceptable des compositions protectrices de la couleur selon l'invention peut par exemple être constitué par de l'eau ou par un mélange d'eau et d'au moins un solvant organique cosmétiquement acceptable. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol et leurs mélanges.

Les solvants sont, de préférence, présents dans des proportions de préférence allant de1 à 40 % en poids environ par rapport au poids total de la composition, et encore plus préférentiellement de 3 à 30 % en poids environ.

Les corps gras insaturés de formule générale (I) peuvent être présents dans les compositions protectrices de la couleur dans des concentrations allant de préférence de 1 à 100% en poids et plus préférentiellement de 5 à 90% en poids par rapport au poids total de la composition.

Selon une forme particulière de l'invention, on utilisera une composition constituée de 100% en poids de corps gras insaturé de formule (I). Dans ce cas, le milieu physiologiquement acceptable est constitué par le ou les corps gras insaturés de formule (I).

La composition selon l'invention contenant l'agent ou les agents protecteurs de la couleur des fibres kératiniques peut également renfermer divers adjuvants utilisés classiquement dans les compositions de traitement capillaire, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Selon une forme préférée de l'invention, les compositions selon l'invention peuvent comprendre en plus au moins un agent protecteur contre les effets des agents atmosphériques notamment la lumière.

Les agents protecteurs des fibres kératiniques peuvent être tout agent actif utile pour prévenir ou limiter les dégradations des fibres kératiniques en particulier des cheveux dues aux agressions atmosphériques et plus particulièrement de la lumière.

Ainsi, l'agent protecteur des fibres kératiniques peut être choisi parmi les filtres UV organiques, les agents anti-radicalaires et les agents antioxydants.

Par "agent anti-radicalaire", on entend tout composé capable de piéger les radicaux libres.

Les filtres UV organiques (systèmes filtrant les radiations UV) sont notamment choisis parmi les filtres hydrosolubles ou liposolubles, siliconés ou non siliconés.

Les filtres organiques sont notamment choisis parmi les dérivés de dibenzoylméthane ; les anthranilates ; les dérivés cinnamiques ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres UV organiques, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
Diisopropyl Methylcinnamate,
Glyceryl Ethylhexanoate Dimethoxycinnamate

### Dérivés du dibenzoylméthane :

Butyl Methoxydibenzoylmethane vendu notamment sous le nom commercial « PARSOL 1789 » par HOFFMANN LA ROCHE,
Isopropyl Dibenzoylmethane vendu notamment sous le nom commercial « EUSOLEX 8020 » par MERCK,

### Dérivés salicyliques :

Homosalate vendu sous le nom « Eusolex HMS » par Rona/EM Industries,
Ethylhexyl Salicylate vendu sous le nom « NEO HELIOPAN OS » par HAARMANN et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés de triazine :

Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,
la 2,4,6-tris-(4'-amino benzalmalonate de diisobutyle)-s- triazine.

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par HOFFMANN LA ROCHE

### Dérivés de 4,4-diarylbutadiène :

-1,1-dicarboxy (2,2'-diméthyl-propyl)-4,4-diphénylbutadiène

### Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V et leurs mélanges.

Comme filtres UV organiques liposolubles (ou lipophiles) convenant à une mise en oeuvre dans la présente invention, on peut citer plus particulièrement :
Ethylhexyl Methoxycinnamate
Butyl Methoxydibenzoylmethane
Homosalate
Ethylhexyl Salicylate,
Octocrylene,
Benzophenone-3,
2-(4-diethylamino-2-hydroxybenzoyl)-benzoate de n-hexyle.
4-Methylbenzylidene camphor,
Ethylhexyl triazone,
Bis-Ethylhexyloxyphenol Methoxyphenyl Triazine
Diethylhexyl Butamido Triazone,
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine

Comme filtres UV organiques hydrosolubles (ou hydrophiles) convenant à une mise en oeuvre dans la présente invention, on peut citer plus particulièrement :
PABA,
PEG-25 PABA
Benzylidene Camphor Sulfonic Acid,
Camphor Benzalkonium Methosulfate
Terephthalylidene Dicamphor Sulfonic Acid
Phenylbenzimidazole Sulfonic Acid
Disodium Phenyl Dibenzimidazole Tetra-sulfonate
Benzophenone-4
Benzophenone-5

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase et les extraits de germes de blé en contenant, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; la guanosine ; les lignanes ; et la mélatonine.

Les agents antioxydants sont notamment choisis parmi les phénols tels que le BHA (tert-butyl-4-hydroxyanisole), le BHT (2,6-di-tert-butyl-p-crésol), le TBHQ (tertiobutylhydroquinone), les polyphénols tels que les oligomères proanthocyanidoliques et les flavonoïdes, les amines encombrées connues sous le vocable générique de HALS (Hindered Amine Light Stabilizer) telles que la tétraaminopipéridine, l'acide érythorbique, les polyamines telles que la spermine, la cystéine, le glutathion, la superoxyde dismutase, la lactoferrine.

Les agents protecteurs des fibres kératiniques vis-à-vis des agressions atmosphériques comme la lumière seront plus particulièrement choisis parmi les filtres UV organiques.

Selon l'invention, le ou les agents protecteurs des fibres kératiniques vis-à-vis des agressions atmosphériques comme la lumière représentent de 0,2 à 20% en poids par rapport au poids total de la composition.

Selon une forme préférée de l'invention, les compositions selon l'invention peuvent comprendre en plus au moins un alcool aromatique et au moins un acide aromatique carboxylique.

Par l'expression "alcool aromatique", on entend tout composé liquide à température ambiante et pression atmosphérique comprenant au moins un cycle benzénique ou naphtalénique et au moins une fonction alcool (OH) directement liée au cycle ou liée sur au moins un substituant dudit cycle. De préférence, la fonction alcool sera sur un substituant du cycle benzénique ou naphtalénique.

Parmi les alcools aromatiques utilisables dans la composition selon l'invention, on peut citer en particulier
l'alcool benzylique
le benzoyl isopropanol
le benzylglycol
le phénoxyéthanol
l'alcool di-chloro-benzylique
le méthylphenylbutanol
le phénoxyisopropanol
le phénylisohexanol
le phénylpropanol
l'alcool phényléthylique
leurs mélanges.

On choisira plus particulièrement l'alcool benzylique.

Selon l'invention, le ou les alcools aromatiques peuvent représenter de 0,01 % à 50 % en poids, de préférence de 0,1 % à 30% en poids et plus particulièrement de 1 à 20% en poids par rapport au poids total de la composition. De préférence, ils seront utilisés à des concentrations supérieures à 1 % en poids.

Les compositions capillaires selon l'invention peuvent comprendre au moins un acide carboxylique aromatique éventuellement salifié.

Par l'expression "acide carboxylique aromatique", on entend tout composé comprenant au moins un cycle benzènique ou naphtalénique et au moins une fonction acide carboxylique (COOH), sous forme libre ou salifiée, directement liée au cycle ou liée sur au moins un substituant dudit cycle. De préférence, la fonction acide sera directement reliée au cycle benzènique ou naphtalénique.

Les sels des acides carboxyliques aromatiques peuvent être choisis notamment parmi les sels de métal alcalin (sodium, potassium), de métal alcalino-terreux (calcium, magnésium) ou les sels d'amines organiques ou d'ammonium.

Parmi les acides carboxyliques aromatiques utilisables dans la composition selon l'invention, on peut citer en particulier
l'acide benzoïque
l'acide para-anisique
l'acide diphénolique
l'acide férulique
l'acide hippurique
l'acide 3-hydroxybenzoique
l'acide 4-hydroxybenzoique
l'acide phénylthioglycolique
l'acide acétylsalicylique
l'acide para, méta ou ortho-phtalique ainsi que leurs formes salifiées et leurs mélanges.

On choisira plus particulièrement l'acide benzoïque.

Selon l'invention, le ou les acides aromatiques ou leurs sels peuvent représenter de 0,001 % à 30 % en poids, de préférence de 0,01 % à 20% en poids et plus particulièrement de 0,1 à 10% en poids par rapport au poids total de la composition.

Les compositions selon l'invention peuvent contenir en plus un ou plusieurs agents de conditionnement.

Dans le cadre de la présente invention, on entend par « agent conditionneur » tout agent ayant pour fonction l'amélioration des propriétés cosmétiques des cheveux, en particulier la douceur, le démêlage, le toucher, le lissage, l'électricité statique.

Les agents de conditionnement peuvent se présenter sous forme liquide, semi-solide ou solide tels que par exemple des huiles, des cires ou des gommes.

Selon l'invention, les agents conditionneurs peuvent être choisis parmi les huiles de synthèses telles que les poly-oléfines, les huiles végétales, les huiles fluorées ou perfluorées, les cires naturelles ou synthétiques, les silicones, les polymères cationiques non polysaccharides, les composés de type céramide, les tensioactifs cationiques, les amines grasses, les acides gras saturés ou les esters d'acides gras autres que ceux de l'invention ainsi que les mélanges de ces différents composés.

Les huiles de synthèse sont notamment les polyoléfines en particulier les poly-α-oléfines et plus particulièrement :
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.
   A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.

De tels produits sont vendus par exemple sous les dénominations ETHYLFLO par la société ETHYL CORP., et d'ARLAMOL PAO par la société ICI.

Les huiles animales ou végétales sont choisies préférentiellement dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur saturé comportant de 7 à 29 atomes de carbone et R₁₀ représente une chaîne hydrocarbonée linéaire ou ramifiée contenant de 3 à 30 atomes de carbone en particulier alkyle ou alkényle, par exemple, l'huile de Purcellin .

On peut également utiliser les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre 1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les agents conditionneurs préférés selon l'invention sont les polymères cationiques et les silicones.

Les polymères cationiques non saccharidiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519863.

Par polymères non saccharides, on entend les polymères ne contenant pas de liaison glycoside entre des monosaccharides.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 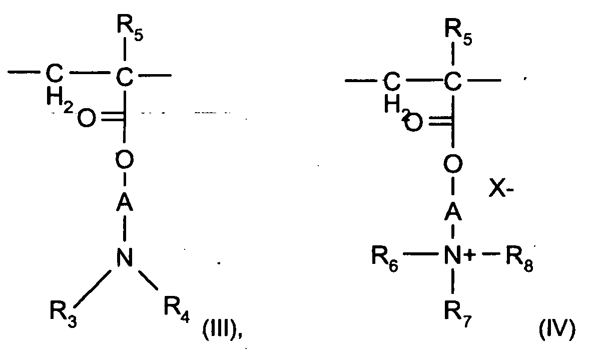 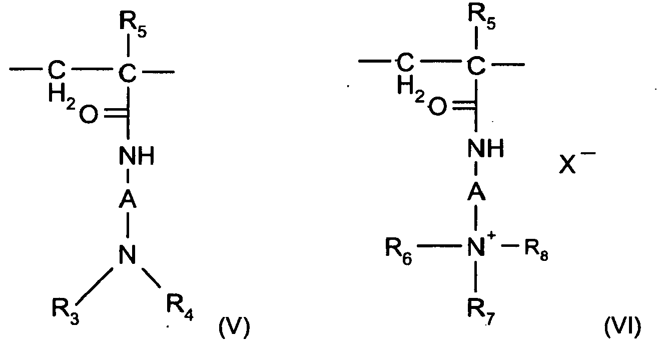 dans lesquelles:
   R₃ et R₄ , identiques ou différents, représentent hydrogène ou un groupe alkyle ayant de
   1 à 6 atomes de carbone et de préférence méthyle ou éthyle;
   R₅ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₆, R₇, R₈, identiques ou différents, représentent un groupe alkyle ayant de 1 à 18 atomes de carbone ou un radical benzyle et de préférence un groupe alkyle ayant de 1 à 6 atomes de carbone;
   X désigne un anion dérivé d'un acide minéral ou organique tel que un anion méthosulfate ou un halogénure tel que chlorure ou bromure.
   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylaminopropyle quaternisé tel que le produit vendu sous la dénomination "GAFQUAT HS 100" par la société ISP.
(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipiquediacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(6) les cyclopolymères d'alkyl diallyl amine ou de dialkyl diallyl ammonium tels que les homopolymères ou copolymères comportant comme constituant principal de la chaîne des motifs répondant aux formules (VII) ou (VIII) : 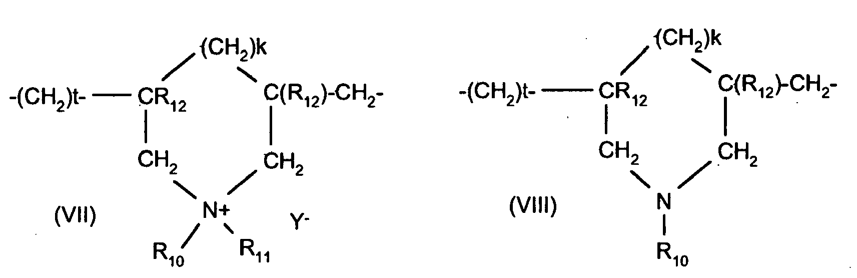 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "Merquat 100" par la société NALCO (et ses homologues de faibles masses moléculaires moyenne en poids) et les copolymères de chlorure de diallyldiméthylammonium et d'acrylamide commercialisés sous la dénomination "MERQUAT 550".
(7) le polymère de diammonium quaternaire contenant des motifs récurrents répondant à la formule : 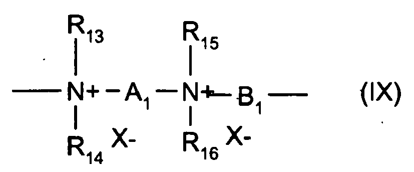 formule (IX) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)ₙₚ-CO-D-OC-(CH₂)p-p est un nombre entier variant de 2 à 20 environ
   dans lequel D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)ₓ -CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂-CH₂-S-S-CH₂-CH₂- ;
   d) un groupement uréylène de formule : -NH-CO-NH- ;
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 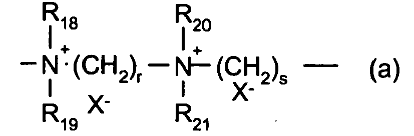 dans laquelle R₁₈, R₁₉, R₂₀ et R₂₁, identiques ou différents, désignent un radical alkyle ou hydroxyalkyle ayant de 1 à 4 atomes de carbone environ, r et s sont des nombres entiers variant de 2 à 20 environ et, X- est un anion dérivé d'un acide minéral ou organique.
   Un composé de formule (a) particulièrement préféré est celui pour lequel R₁₈, R₁₉, R₂₀ et R21, représentent un radical méthyle et r = 3, s = 6 et X = Cl, dénommé Hexadimethrine chloride selon la nomenclature INCI (CTFA).
(8) les polymères de polyammonium quaternaires constitués de motifs de formule (X): 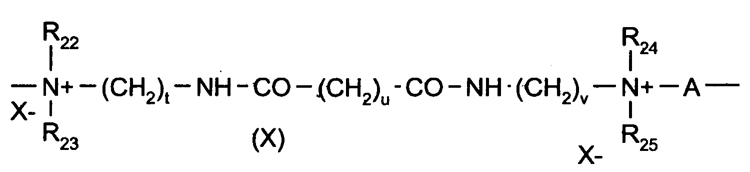 formule dans laquelle :
   R₂₂, R₂₃, R₂₄ et R₂₅, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₂₂, R₂₃, R₂₄ et R₂₅ ne représentent pas simultanément un atome d'hydrogène,
   t et u, identiques ou différents, sont des nombres entiers compris entre 1 et 6,
   v est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.
   De tels composés sont notamment décrits dans la demande de brevet EP-A-122 324. On peut par exemple citer parmi ceux-ci, les produits "Mirapol® A 15", "Mirapol® AD1", "Mirapol® AZ1" et "Mirapol® 175" vendus par la société Miranol.
(9) Les polymères quaternaires de vinylpyrrolidone et de vinylimidazole tels que par exemple les produits commercialisés sous les dénominations Luviquat® FC 905, FC 550 et FC 370 par la société B.A.S.F.
(10) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE^{®} SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE^{®} SC 95 » et « SALCARE^{®} SC 96 » par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole et leurs mélanges.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 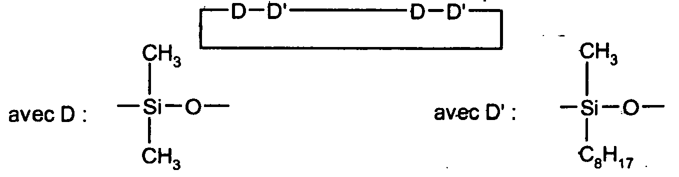 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHODIA CHIMIE telles que par exemple l'huile 70 047 V 500 000 ;
- les huiles de la série MIRASIL commercialisées par la société RHODIA CHIMIE ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE de la série 70 641 de RHODIA CHIMIE ;
- les huiles des séries RHODORSIL 70 633 et 763 de RHODIA CHIMIE ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (XI) : 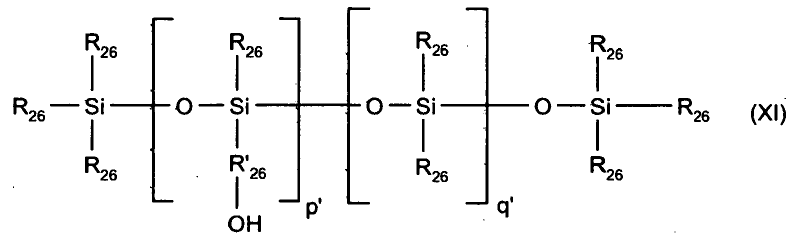
dans laquelle les radicaux R₂₆ identiques ou différents sont choisis parmi les radicaux méthyle et phényle ; au moins 60 % en mole des radicaux R₂₆ désignant méthyle ; le radical R'₂₆ est un chaînon alkylène divalent hydrocarboné en C₂-C₁₈ ; p' est compris entre 1 et 30 inclus ; q' est compris entre 1 et 150 inclus ;
- des groupements acyloxyalkyle tels que par exemple les polyorganosiloxanes décrits dans le brevet US-A-4957732 et répondant à la formule (XII) : 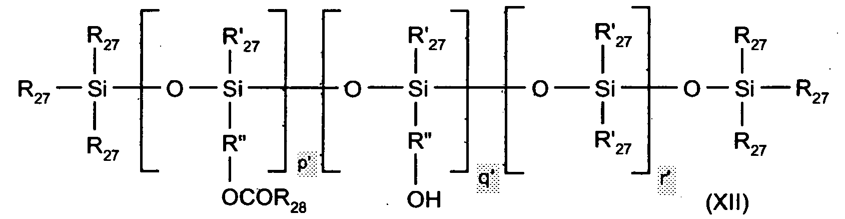
dans laquelle :
R₂₇ désigne un groupement méthyle, phényle, -OCOR₂₈, hydroxyle, un seul des radicaux R₂₇ par atome de silicium pouvant être OH ;
R'₂₇ désigne méthyle, phényle ; au moins 60 % en proportion molaire de l'ensemble des radicaux R₄ et R'₄ désignant méthyle ;
R₂₈ désigne alkyle ou alcényle en C₈-C₂₀ ;
R" désigne un radical alkylène hydrocarboné divalent, linéaire ou ramifié en C₂-C₁₈ ;
r' est compris entre 1 et 120 inclus ;
p' est compris entre 1 et 30 ;
q' est égal à 0 ou est inférieur à 0,5 p', p' + q' étant compris entre 1 et 30 ; les polyorganosiloxanes de formule (XII) peuvent contenir des groupements : 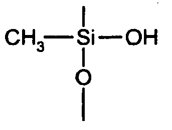
dans des proportions ne dépassant pas 15 % de la somme p + q + r.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412704, EP-A-412707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 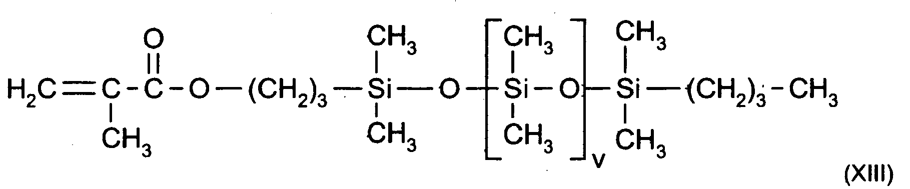
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de micrémulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHODIA CHIMIE, les polyalkylsiloxanes à groupements terminaux diméthylsilanol tels que les diméthiconol ou les polyalkylarylsiloxanes tels que l'huile SILBIONE 70641 V 200 commercialisée par la société RHODIA CHIMIE ;
- la résine d'organopolysiloxane commercialisée sous la dénomination DOW CORNING 593 ;
- les polysiloxanes à groupements aminés tels que les amodiméthicones ou les triméthylsilylamodiméthicone ;

Les protéines ou hydrolysats de protéines cationiques sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société MAYBROOOK et dénommés dans le dictionnaire CTFA "Steartrimonium Hydrolyzed Collagen" ;
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le "Croquat L" dont les groupements ammonium quaternaires comportent un groupement alkyle en C₁₂ ;
- le "Croquat M" dont les groupements ammonium quaternairees comportent des groupements alkyle en C₁₀-C₁₈ ;
- le "Croquat S" dont les groupements ammonium quaternaires comportent un groupement alkyle en C₁₈ ;
- le "Crotein Q" dont les groupements ammonium quaternaires comportent au moins un groupe alkyle ayant de 1 à 18 atomes de carbone.
   Ces différents produits sont vendus par la Société Croda.

D'autre protéines ou hydrolysats quaternisés sont par exemple ceux répondant à la formule (XIV) : 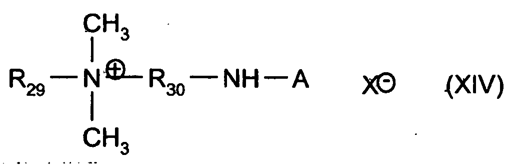 dans laquelle X⁻ est un anion d'un acide organique ou minéral, A désigne un reste de protéine dérivé d'hydrolysats de protéine de collagène, R₂₉ désigne un groupement lipophile comportant jusqu'à 30 atomes de carbone, R₃₀ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Selon la présente invention, Les composés de type céramide sont notamment les céramides et/ou les glycocéramides et/ou les pseudocéramides et/ou les néocéramides, naturelles ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO94/07844, WO94/24097, WO94/10131 dont les enseignements sont ici inclus à titre de référence.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine
ou les mélanges de ces composés.

On peut également utiliser des tensioactifs cationiques parmi lesquels on peut citer en particulier : les sels d'amines grasses primaires, secondaires ou tertiaires, éventuellement polyoxyalkylénées ; les sels d'ammonium quaternaire ; les dérivés d'imidazoline ; ou les oxydes d'amines à caractère cationique.

Les sels d'ammonium quaternaires sont par exemple :
- ceux qui présentent la formule générale (XV) suivante : 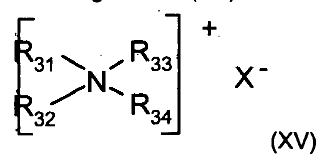 dans laquelle les radicaux R₃₁ à R₃₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium, comme par exemple celui de formule (XVI) suivante : 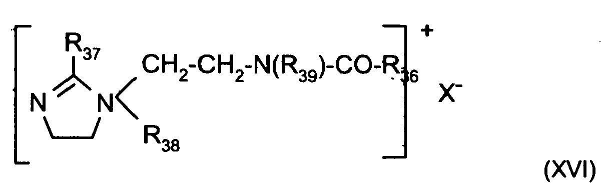
dans laquelle R₃₆ représente un radical alcényle ou alkyle comportant de 8 à 30 atomes de carbone par exemple dérivés des acides gras du suif, R₃₇ représente un atome d'hydrogène, un radical alkyle en C₁-C₄ ou un radical alcényle ou alkyle comportant de 8 à 30 atomes de carbone, R₃₈ représente un radical alkyle en C₁-C₄ , R₃₉ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₃₆ et R₃₇ désignent un mélange de radicaux alcényle ou alkyle comportant de 12 à 21 atomes de carbone par exemple dérivés des acides gras du suif, R₃₈ désigne méthyle, R₃₉ désigne hydrogène. Un tel produit est par exemple commercialisé sous la dénomination «REWOQUAT W 75» par la société DEGUSSA,
- les sels de diammonium quaternaire de formule (XVII) : 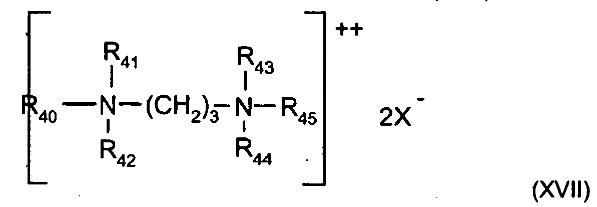 dans laquelle R₄₀ désigne un radical aliphatique comportant environ de 16 à 30 atomes de carbone, R₄₁, R₄₂, R₄₃, R₄₄ et R₄₅ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire contenant au moins une fonction ester

Les sels d'ammonium quaternaire contenant au moins une fonction ester utilisables selon l'invention sont par exemple ceux de formule (XVIII) suivante : 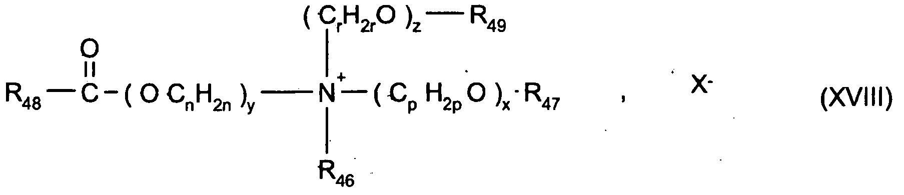 dans laquelle :
- R₄₆ est choisi parmi les radicaux alkyles en C₁-C₆ et les radicaux hydroxyalkyles ou dihydroxyalkyles en C₁-C₆ ;
- R₄₇ est choisi parmi :
- le radical 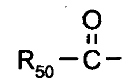
- les radicaux R₅₁ hydrocarbonés en C₁-C₂₂ linéaires ou ramifiés, saturés ou insaturés,
- l'atome d'hydrogène,
- R₄₉ est choisi parmi :
- le radical 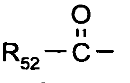
- les radicaux R₅₃ hydrocarbonés en C₁-C₆ linéaires ou ramifiés, saturés ou insaturés,
- l'atome d'hydrogène,
- R₄₈, R₅₀ et R₅₂, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;
sous réserve que la somme x + y + z vaut de 1 à 15 , que lorsque x vaut 0 alors R₄₇ désigne R₅₁ et que lorsque z vaut 0 alors R₄₉ désigne R₅₃.

Les radicaux alkyles R₄₆ peuvent être linéaires ou ramifiés et plus particulièrement linéaires.

De préférence R₄₆ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₄₇ est un radical R₅₁ hydrocarboné, il peut être long et avoir de 12 à 22 atomes de carbone ou court et avoir de 1 à 3 atomes de carbone.

Lorsque R₄₉ est un radical R₅₃ hydrocarboné, il a de préférence 1 à 3 atomes de carbone.

Avantageusement, R₄₈, R₅₀ et R₅₂, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement les sels d'ammonium de formule (XVIII) dans laquelle :
- R₄₆ désigne un radical méthyle ou éthyle,
- x et y sont égaux à 1 ;
- z est égal à 0 ou 1 ;
- n, p et r sont égaux à 2 ;
- R₄₇ est choisi parmi :
- le radical 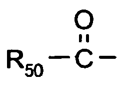
- les radicaux méthyle, éthyle ou hydrocarbonés en C₁₄-C₂₂
- l'atome d'hydrogène ;
- R₄₉ est choisi parmi :
- le radical 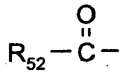
- l'atome d'hydrogène ;

R₄₈, R₅₀ et R₅₂, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.
Avantageusement, les radicaux hydrocarbonés sont linéaires.

On peut citer par exemple les composés de formule (XVI) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme
ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société COGNIS, STEPANQUAT par la société STEPAN, NOXAMIUM par la société CECA, REWOQUAT WE 18 par la société DEGUSSA.

On peut aussi utiliser les sels d'ammonium contenant au moins une fonction ester décrits dans les brevets US-A-4874554 et US-A-4137180.

Parmi les sels d'ammonium quaternaire de formule (XV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Les acides gras saturés sont choisis plus particulièrement parmi l'acide myristique, l'acide palmitique, l'acide stéarique, l'acide béhénique, et l'acide isostéarique.

Les esters d'acides gras sont notamment les esters d'acides carboxyliques en particulier les esters mono, di, tri ou tétracarboxyliques.

Les esters d'acides monocarboxyliques sont différents de ceux de l'invention et sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle, le néopentanoate d'isostéaryle, le néopentanoate d'isodécyle.

On peut également utiliser les esters d'acides di ou tricarboxyliques en C4-C22 et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle, le néopentanoate d'isostéaryle, le néopentanoate d'isodécyle.

Les huiles fluorées sont par exemple les perfluoropolyéthers décrits notamment dans la demande de brevet EP-A-486135 et les composés fluorohydrocarbonées décrites notamment dans la demande de brevet WO 93/11103. L'enseignement de ces deux demandes est totalement inclus dans la présente demande à titre de référence.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Il est bien entendu possible de mettre en oeuvre des mélanges d'agents conditionneurs.

Selon l'invention, le ou les agents conditionneurs peuvent représenter de 0,001 % à 20 % en poids, de préférence de 0,01 % à 10% en poids et plus particulièrement de 0,1 à 3% en poids par rapport au poids total de la composition finale.

Les compositions protectrices de la couleur des fibres kératiniques selon l'invention peuvent se présenter sous forme de lotions aqueuses ou hydroalcooliques pour le soin des cheveux. Les compositions cosmétiques selon l'invention peuvent également se présenter sous forme d'huile, de gel, de lait, de crème, d'émulsion ou de mousse.

Les compositions protectrices de la couleur des fibres kératiniques peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Le pH de la composition protectrice des fibres kératiniques varie généralement de 1 à 11. Il est de préférence de 2 à 6, et peut être ajusté à la valeur désirée au moyen d'agents acidifiants ou alcalinisants bien connus de l'état de la technique des compositions appliquées sur des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule suivante : 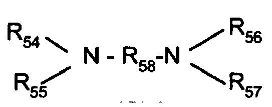 dans laquelle R₅₈ est un reste propylène éventuellement substitué par un groupement hydroxyle ou un radical alkyle en C₁-C₄ ; R₅₄, R₅₅, R₅₆ et R₅₇, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

L'invention a donc pour objet un procédé pour protéger vis-à-vis du lavage la couleur des fibres kératiniques teintes artificiellement, caractérisé par le fait qu'il consiste à appliquer sur lesdites fibres, avant ou après teinture, au moins une composition comprenant dans un milieu cosmétiquement acceptable au moins un corps gras insaturé de formule (I) tel que défini précédemment.

De manière préférentielle, la composition comprenant le corps gras insaturé de formule (I) sera appliquée sur lesdites fibres après l'étape de teinture.

Il est aussi possible de prévoir, une étape de rinçage et/ou une étape de lavage au shampoing avant ou après l'application de la composition contenant le ou les corps gras insaturés de formule (I).

Le procédé selon l'invention peut comprendre une étape supplémentaire de séchage total ou partiel des fibres kératiniques avec un séchoir.

Selon une forme particulière de l'invention, le procédé de protection de la couleur des fibres kératiniques peut comprendre une étape de chauffage de la composition comprenant le ou les corps gras insaturé de formule (I) que l'on appliquera ensuite directement sur les fibres kératiniques. La température sera de préférence inférieure ou égale à 70°C.

Selon une forme particulière de l'invention, le procédé de protection de la couleur des fibres kératiniques peut comprendre une étape de chauffage des fibres kératiniques après application de la composition comprenant le ou les corps gras insaturé de formule (I).

Le chauffage des fibres kératiniques peut par exemple être effectué au moyen d'un fer, d'un mélange eau liquide/vapeur d'eau ou bien au moyen d'un casque chauffant.

Le fer chauffant utile dans le cadre de l'invention est un fer chauffant conventionnellement utilisé dans le domaine capillaire. Un tel fer, par exemple un fer à friser ou un fer à lisser, est bien connu dans le domaine du traitement capillaire. Par exemple, des fers utiles pour mettre en oeuvre la présente invention sont des fers plats ou ronds décrits dans les brevets US 4 103 145, US 4 308 878, US 5 983 903, US 5 957 140, US 5 494 058. L'application du fer peut se faire par touches séparées successives de quelques secondes, ou par déplacement ou glissement progressif le long des mèches. Il est possible entre l'application de la composition protectrice de la couleur et l'application du fer chauffant sur les fibres kératiniques de prévoir un temps de pause. Ledit temps de pause de préférence variera de 30 secondes à 60 minutes et plus préférentiellement de 1 à 30 minutes. La température va de préférence de 60°C à 120°C.

Le mélange eau liquide/vapeur d'eau utile dans le cadre de l'invention a en général une température d'au moins 35°C.

Le mélange eau liquide/vapeur d'eau constitue une brume. Ledit mélange peut contenir en plus au moins un autre gaz tel que l'oxygène ou l'azote, des mélanges de gaz tels que l'air ou bien encore d'autres composés vaporisables.

La température du mélange eau liquide/vapeur d'eau est de préférence supérieure ou égale à 40°C, et est plus particulièrement comprise entre 40°C et 75°C environ.

De préférence, le mélange eau liquide/vapeur d'eau est mis en contact avec la fibre pendant une durée allant de 1 seconde à 1 heure, et encore plus préférentiellement de 5 minutes à 15 minutes. Bien entendu, l'application dudit mélange peut être répétée plusieurs fois sur la même fibre, chaque opération se faisant selon une durée telle qu'indiquée ci-dessus. Plus préférentiellement, on applique tout d'abord sur les cheveux la composition contenant les composés de formule (I), puis on soumet ces mèches ainsi imprégnées à l'action du mélange eau liquide/vapeur d'eau selon les conditions mentionnées précédemment, puis on refroidit les mèches ainsi traitées par exemple en envoyant sur ou à travers celles-ci un courant d'air froid ou d'air à température ambiante.

La production du mélange eau liquide/vapeur d'eau utilisé selon l'invention peut se faire à l'aide de tout appareil connu en soi et prévu à cet effet. Toutefois, selon la présente invention, on utilise de préférence un appareil comprenant au moins un générateur de vapeur d'eau directement relié à un casque diffusant sur les fibres kératiniques en particulier les cheveux humains le mélange eau liquide/vapeur d'eau. Comme type d'appareil, on utilisera plus particulièrement celui vendu sous le nom ®MICROMIST par la Société TAKARA BELMONT.

Un autre objet de l'invention concerne un procédé de coloration consistant à appliquer sur les fibres kératiniques humaines et plus particulièrement des cheveux, une composition (A) colorante directe ou d'oxydation pendant un temps suffisant pour développer la couleur, et de faire suivre ou précéder cette application par l'application d'une composition (B) contenant dans un milieu cosmétiquement acceptable au moins un corps gras insaturé de formule (I) telle que définie précédemment.

L'application de la composition (A) peut être suivie d'un rinçage et/ou d'un séchage des fibres kératiniques.

L'application de la composition (B) peut être suivie d'un rinçage et/ou d'un séchage des fibres kératiniques. La composition (B) peut être préalablement chauffée dans les mêmes conditions définies ci-dessus. L'application de la composition (B) peut être suivie par un chauffage des fibres kératiniques dans les mêmes conditions définies ci-dessus.

De manière préférentielle, la composition (B) sera appliquée après l'application de la composition (A) colorante directe ou d'oxydation. La composition (B) comprenant le ou les corps gras insaturés de formule (I) peut être appliquée immédiatement après coloration ou de manière différée. Par différée, on entend une application se faisant quelques heures, un jour ou plusieurs jours (de 1 à 15 jours) après la coloration. De préférence, la composition (B) sera appliquée immédiatement après coloration des fibres kératiniques. ; les applications de ladite composition pouvant être répétées entre deux colorations.

La nature et la concentration des colorants présents dans la composition (A) colorante n'est pas critique.

Dans le cas des colorations directes éclaircissantes les compositions colorantes (A) résultent du mélange au moment de l'emploi d'une composition colorante (A₁) contenant au moins un colorant direct et d'une composition (A₂) contenant un agent oxydant.

Dans le cas des colorations d'oxydation, les compositions colorantes (A) résultent du mélange au moment de l'emploi d'une composition colorante (A₃) contenant au moins une base d'oxydation et éventuellement au moins un coupleur et/ou un colorant direct et d'une composition (A₄) contenant un agent oxydant.

Les colorants directs sont plus particulièrement des composés absorbant les radiations lumineuses dans le domaine visible (400-750 nm). Ils peuvent être de nature non ionique, anionique ou cationique.

Généralement, les colorants directs sont choisis parmi les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ceux dérivés du triarylméthane, seuls ou en mélanges.

Parmi les colorants benzéniques nitrés, on peut citer les composés rouges ou orangés suivants : le 1-hydroxy-3-nitro-4-N-(γ-hydroxypropyl)amino benzène, le N-(β-hydroxy éthyl)amino-3-nitro-4-amino benzène, le 1-amino-3-méthyl-4-N-(β-hydroxyéthyl)amino-6-nitro benzène, le 1-hydroxy-3-nitro-4-N-(β-hydroxyéthyl)-amino benzène, le 1,4-diamino-2-nitrobenzène, le 1-amino-2-nitro-4-méthylamino benzène, la N-(β-hydroxyéthyl)-2-nitro-paraphénylènediamine, le 1-amino-2-nitro-4-(β-hydroxy éthyl)amino-5-chloro benzène, la 2-nitro-4-amino-diphénylamine, le 1-amino-3-nitro-6-hydroxybenzène, le 1-(β-amino éthyl)amino-2-nitro-4-(β-hydroxy éthyloxy) benzène, le 1-(β,γ-dihydroxypropyl)oxy-3-nitro-4-(β-hydroxyéthyl)amino benzène, le 1-hydroxy-3-nitro-4-aminobenzène, le 1-hydroxy-2-amino-4,6-dinitrobenzène, le 1-méthoxy-3-nitro-4-(β -hydroxyéthyl)amino benzène, la 2-nitro-4'-hydroxydiphénylamine, le 1-amino-2-nitro-4-hydroxy-5-méthylbenzène, seuls ou en mélanges.

En ce qui concerne les colorants directs benzéniques nitrés, on peut mettre en oeuvre des colorants de ce type jaunes et jaune-verts, comme par exemple le 1-β-hydroxyéthyloxy-3-méthylamino-4-nitrobenzène, le 1-méthylamino-2-nitro-5-(β, γ-dihydroxypropyl)oxy benzène, le 1-(β-hydroxyéthyl)amino-2-méthoxy-4-nitrobenzène, le 1-(β-amino éthyl)amino-2-nitro-5-méthoxy-benzène, le 1,3-di(β-hydroxyéthyl)amino-4-nitro-6-chloro benzène, le 1-amino-2-nitro-6-méthyl-benzène, le 1-(β-hydroxyéthyl)amino-2-hydroxy-4-nitrobenzène, la N-(β-hydroxyéthyl)-2-nitro-4-trifluorométhylaniline, l'acide 4-( β-hydroxy éthyl)amino-3-nitro-benzènesulfonique, l'acide 4-éthylamino-3-nitro-benzoïque, le 4-(β-hydroxyéthyl)amino-3-nitro-chiorobenzène, le 4-(β-hydroxyéthyl)amino-3-nitrométhyl benzène, le 4-(β,γ-dihydroxypropyl)amino-3-nitro-trifluorométhylbenzène, le 1-(β-uréido éthyl)amino-4-nitrobenzène, le 1,3-diamino-4-nitrobenzène, le 1-hydroxy-2-amino-5-nitrobenzène, le 1-amino-2-[tris(hydroxyméthyl)méthyl]amino-5-nitro-benzène, le 1-(β-hydroxyéthyl)amino-2-nitrobenzène, le 4-(β-hydroxyéthyl)amino-3-nitrobenzamide.

Il est de même envisageable d'utiliser des colorants benzéniques nitrés bleus ou violets, comme entre autres le 1-(β-hydroxyéthyl)amino-4-N,N-bis-(β-hydroxyéthyl)amino 2-nitrobenzène, le 1-(γ -hydroxypropyl)amino 4-N,N-bis-(β -hydroxyéthyl)amino 2-nitrobenzène, le 1-(β -hydroxyéthyl)amino 4-(N-méthyl, N- β -hydroxyéthyl)amino 2-nitrobenzène, le 1-(β -hydroxyéthyl)amino 4-(N-éthyl, N- β -hydroxyéthyl)amino 2-nitrobenzène, le 1-(β,γ-dihydroxypropyl)amino 4-(N-éthyl, N-β-hydroxyéthyl)amino 2-nitrobenzène, les 2-nitroparaphénylènediamines de formule suivante : 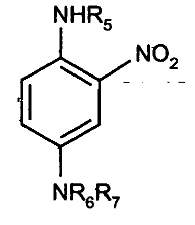 dans laquelle :
- R₆ représente un radical alkyle en C1-C4, un radical β-hydroxyéthyle ou β-hydroxypropyle ou γ-hydroxypropyle ;
   R₅ et R₇, identiques ou différents, représentent un radical β-hydroxyéthyle, β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans le brevet français FR 2 692 572.

Il est rappelé que les colorants azoïques sont des composés comportant dans leur structure au moins un enchaînement -N=N- non inclus dans un cycle ; les colorants méthiniques sont des composés comportant dans leur structure au moins un enchaînement -C=C- non inclus dans un cycle ; les colorants azométhinique sont des composés comportant dans leur structure au moins un enchaînement -C=N- non inclus dans un cycle.

Les colorants dérivés de triarylméthane comportent dans leur structure au moins un enchaînement suivant :

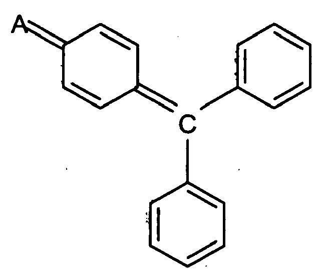

### A désignant un atome d'oxygène ou d'azote

Les colorants xanthéniques comportent dans leur structure au moins un enchaînement de formule :

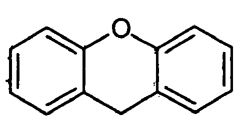

Les colorants phénanthridiniques comportent dans leur structure au moins un enchaînement de formule

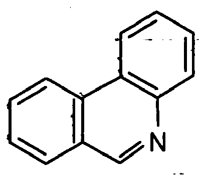

Les colorants phtalocyanines comportent dans leur structure au moins un enchaînement de formule :

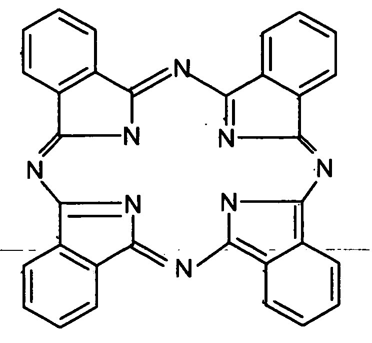

Les colorants phénotiaziniques comportent dans leur structure au moins un enchaînement suivant :

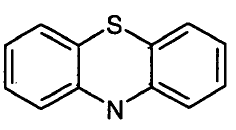

Les colorants directs peuvent de plus être choisis parmi les colorants basiques comme ceux listés dans le Color Index, 3ème édition, notamment sous les dénominations "Basic Brown 16", "Basic Brown 17", "Basic Yellow 57", "Basic Red 76", "Basic Violet 10", "Basic Blue 26" et "Basic Blue 99" ; ou parmi les colorants directs acides, listés le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore les colorants directs cationiques tels que ceux décrits dans les demandes de brevet WO 95/01772, WO 95/15144 et EP 714954 et en particulier « Basic Red 51 », « Basic Orange 31 », « Basic Yellow 87 ».

Lorsqu'ils sont présents, le ou les colorants directs représentent de préférence de 0,0005 à 12 % en poids environ du poids total de la composition tinctoriale, et encore plus préférentiellement de 0,005 à 6 % en poids environ de ce poids.

Les bases d'oxydation peuvent être choisies parmi les bases d'oxydation classiquement utilisées en teinture d'oxydation et parmi lesquelles on peut notamment citer les paraphénylènediamines , les bis-phénylalkylènediamines, les paraaminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyt)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]-pyrimidin-7-yl)-(2-hydroxy-éthyl)-amino]-éthanol, le 2-[(7-amino-pyrazolo[1,5-a]-pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1 ,5-a]-pyrimidine et leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols, les naphtols et les coupleurs hétérocycliques tels que par exemple les dérivés indoliques, les dérivés indoliniques, les dérivés pyridiniques, les dérivés indazoliques, les dérivés de pyrazolo[1,5-b]-1,2,4-triazole, les dérivés de pyrazolo[3,2-c]-1,2,4-triazole, les dérivés de benzimidazole, les dérivés de benzothiazole, les dérivés de benzoxazole, les dérivés de 1,3-benzodioxole et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthyiamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 6-hydroxybenzomorpholine, la 3,5 diamino 2,6-diméthoxy pyridine, le 1-N-(β hydroxyéthyl)amino 3,4 méthylènedioxy benzène, le 2,6 bis(β hydroxyéthylamino)toluène, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La nature de l'agent oxydant utilisé dans la coloration directe éclaircissante (coloration directe avec un agent oxydant) ou dans la coloration d'oxydation n'est pas critique.

L'agent oxydant est de préférence choisi dans le groupe formé par le peroxyde d'hydrogène, le peroxyde d'urée, les bromates ou ferricyanures de métaux alcalins, les persels tels que les perborates et les persulfates. On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Selon un mode particulier de l'invention, on peut utiliser le procédé de l'invention sur des cheveux sensibilisés par des traitements capillaires autres que ceux de l'invention cités précédemment.

L'invention a également pour objet un agent de coloration multi-composants ou kit comportant au moins un premier composant comprenant une composition (A) de coloration directe et un deuxième composant comprenant une composition (B) contenant dans un milieu cosmétiquement acceptable, au moins un corps gras insaturé de formule (I) telle que définie ci-dessus.

L'invention a également pour objet un agent de coloration multi-composants ou kit comportant au moins un premier composant comprenant une composition (A₁) comprenant au moins un colorant direct, un deuxième composant comprenant une composition (A₂) contenant au moins un agent oxydant et un troisième composant comprenant une composition (B) contenant dans un milieu cosmétiquement acceptable, au moins un corps gras insaturé de formule (I) telle que définie ci-dessus.

L'invention a également pour objet un agent de coloration multi-composants ou kit comportant au moins un premier composant comprenant une composition (A₃) contenant au moins une base d'oxydation et éventuellement au moins un coupleur et/ou un colorant direct, un deuxième composant comprenant une composition (A₄) contenant au moins un agent oxydant et un troisième composant comprenant une composition (B) contenant dans un milieu cosmétiquement acceptable au moins un corps gras insaturé de formule (I) telle que définie ci-dessus.

L'invention va être maintenant plus complètement illustrée à l'aide des exemples suivants qui ne sauraient être considérés comme limitatifs.

### EXEMPLE:

### Etape de coloration :

Au moment de l'emploi, une composition de coloration d'oxydation de support RECITAL ® contenant 6.10⁻⁴ moles % de p-phenylènediamine et 6.10⁻⁴ moles % de 4-amino-2-hydroxytoluene est mélangée avec de l'eau oxygénée (Eau oxygénée l'Oréal professionnel 20 volumes à 6%) poids pour poids.

Le mélange est ensuite appliqué sur des mèches de cheveux 90% blancs permanentées à raison de 10 g de mélange colorant/ g de mèche. Le temps de pause est de 15 minutes de chaque côté de la mèche. Les mèches sont alors rincées à l'eau, puis lavées par le shampooing DOP camomille et séchées.

### Etapes de traitement protecteur :

On applique alors sur une mèche colorée, différents corps gras purs à raison de 2 grammes par gramme de cheveu :

| **Traitement appliqué** | **Mèche** |
|---|---|
| Acide oléique pur commercialisé sous la référence 01383 par Sigma Aldrich | A |
| Acide linoléique pur commercialisé sous la référence L1012 par Sigma Aldrich | B |
| Oléate de sorbitane | C |

Puis, les mèches traitées sont alors laissées pauser 30 mn à 45°C.

Les mèches traitées par subissent alors deux shampooings DOP CAMOMILLE ® de manière à éliminer l'huile résiduelle de surface.

Des mèches non traitées (références) subissent également ces 2 shampooings.
Les mèches sont alors séchées au casque 10mn à 60°C.

### Etapes de ténacité aux lavages:

### Lavages

Une épreuve de ténacité shampooings est réalisée sur les mèches précédentes avec un shampooing DOP CAMOMILLE ®. On réalise jusqu'à 10 shampooings successifs avec séchage intermédiaire.

### Evaluation de la protection de la couleur

La dégradation de la couleur après lavages des mèches traitées et non traitées est évaluée visuellement par rapport à des mèches colorées non lavées.

Un suivi spectro-colorimétrique accompagne ces évaluations. Des mesures sont réalisées à l'aide du spectrocolorimètre MINOLTA CM2022 jusqu'à 10 lavages :
La dégradation provoquée par les lavages est exprimée en ΔE
ΔE(x shampooings - 0 shampooing)=√ (ΔL*² +Δa*² +Δb*²)

On exprime alors la protection par une différence en ΔE entre les mèches traitées et non traitées. (Différence positive=Gain en protection couleur, Différence négative=perte en protection, Différence significative avec un gain en ΔE ≥2)

### Résultats :

Après 10 shampooings, on observe une dégradation importante de la coloration des mèches colorées non traitées (perte en ΔE=12.98).

On observe de façon surprenante, qu'après 10 lavages, seules les mèches qui ont été traitées par des corps gras de l'invention A et B et C apportent une protection significative de la couleur par rapport aux mèches non traitées.

Ces résultats sont confirmés par les mesures colorimétriques qui indiquent un gain en ΔE significatif par rapport à la mèche non traitée.

**Résultats de dégradation de la couleur après épreuve de ténacité de 10 lavages (shampooings DOP CAMOMILLE ®)**

| **Mèche** | **ΔE par rapport aux mèches non lavées** | **Protection significative par rapport à la mèche non traitée** |
|---|---|---|
| A | 10.31 | oui |
| B | 10.11 | oui |
| C | 7.19 | oui |

## Revendications

1. Utilisation d'au moins un corps gras insaturé de formule (I) suivante : 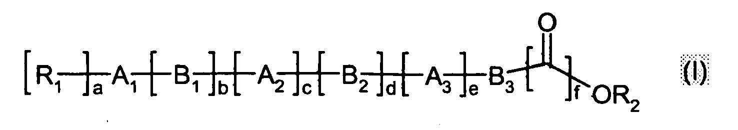 où :
A₁, A₂, A₃ , identiques ou différents, désignent un radical hydrocarboné monovalent ou divalent, linéaire ou cyclique comportant au moins une insaturation ;
B₁, B₂, B₃, identiques ou différents, désignent un radical CₙH₂ₙ où n est un entier inférieur à 20 ;
R₁ désigne un alkyle linéaire ou ramifié en C₁-C₁₂ ;
R₂ désigne hydrogène, un métal alcalin M ou le groupe sorbitane de formule : 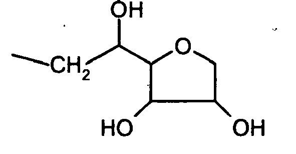 a,b,c, d, e, f , identiques ou différents, valent 0 ou 1,
comme agent permettant de protéger la couleur vis-à-vis du lavage des fibres kératiniques teintes artificiellement, notamment des fibres kératiniques humaines et plus particulièrement des cheveux.

2. Utilisation selon la revendication 1, où les composés de formule (I) sont choisis parmi les composés de formule (II) suivante : 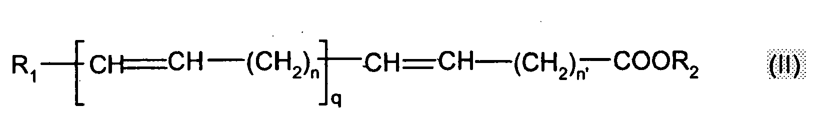 dans laquelle :
R₁ désigne un alkyle linéaire ou ramifié en C₁-C₁₂ ;
R₂ désigne hydrogène, un métal alcalin ou le groupe sorbitane de formule : 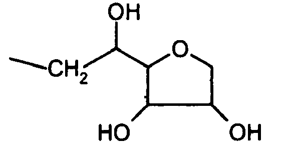
n et n', identiques ou différents, sont des entiers allant de 1 à 10 et de préférence de 1 à 8;
q vaut 0,1 ou 2.

3. Utilisation selon la revendication 1 ou 2, où le composé de formule (I) est choisi parmi :
l'acide lauroléique;
l'acide myristoléique ;
l'acide palmitoléique
l'acide oléique ;
l'acide linoléique ;
l'acide linolénique,
l'oléate de sorbitan

4. Utilisation selon la revendication 3, où le composé de formule (I) est choisi parmi l'acide oléique, l'oléate de sorbitan et l'acide linoléique.

5. Procédé pour protéger la couleur vis-à-vis du lavage des fibres kératiniques teintes artificiellement notamment des fibres kératiniques humaines et plus particulièrement des cheveux, **caractérisé par le fait qu'**il consiste à appliquer sur lesdites fibres, avant ou après teinture, au moins une composition comprenant dans un milieu cosmétiquement acceptable au moins un corps gras insaturé de formule (I) telle que définie selon l'une quelconque des revendications 1 à 4.

6. Procédé selon la revendication 5 selon lequel la composition comprenant dans un milieu cosmétiquement acceptable au moins un corps gras insaturé de formule (I) est appliquée sur les fibres après teinture.

7. Procédé selon la revendication 5 ou 6, où le milieu cosmétiquement acceptable de la composition est constitué par de l'eau ou par un mélange d'eau et d'au moins un solvant organique cosmétiquement acceptable.

8. Procédé selon la revendication 7, où le solvant organique est choisi parmi les alcanols inférieurs en C₁-C₄, tels que l'éthanol et l'isopropanol ; les polyols et éthers de polyols et leurs mélanges.

9. Procédé selon la revendication 7 ou8, où le ou les solvants sont présents dans des proportions allant de 1 à 40 % en poids environ par rapport au poids total de la composition et encore plus préférentiellement de 3 à 30 % en poids environ par rapport au poids total de la composition.

10. Procédé selon l'une quelconque des revendications 5 à 9, où le ou les corps gras insaturés de formule générale (I) sont présents dans des concentrations allant de 1 à 100% en poids et plus préférentiellement de 5 à 90% en poids par rapport au poids total de la composition.

11. Procédé selon l'une quelconque des revendications 5 à 10, où la composition est constituée de 100% en poids de corps gras insaturé de formule (I).

12. Procédé selon l'une quelconque des revendications 5 à 11, où la composition contient au moins un additif choisi parmi des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

13. Procédé selon l'une quelconque des revendications 5 à 12, où la composition contient au moins un agent protecteur contre les effets des agents atmosphériques notamment la lumière.

14. Procédé selon la revendication 13, où l'agent protecteur contre les effets des agent atmosphériques est choisi parmi les filtres UV organiques, les agents anti-radicalaires et les agents antioxydants.

15. Procédé selon l'une quelconque des revendications 5 à 14, où la composition contient au moins un alcool aromatique et au moins un acide aromatique carboxylique.

16. Procédé selon l'une quelconque des revendications 5 à 15, où la composition contient au moins un ou plusieurs agents de conditionnement.

17. Procédé selon la revendication 16, où le ou les agents conditionneurs représentent de 0,001 % à 20 % en poids, de préférence de 0,01 % à 10% en poids et plus particulièrement de 0,1 à 3% en poids par rapport au poids total de la composition.

18. Procédé selon l'une quelconque des revendications 5 à 17, où la composition se présente sous forme de lotion aqueuses ou hydroalcoolique sous forme d'huile, de gel, de lait, de crème, d'émulsion ou de mousse.

19. Procédé selon l'une quelconque des revendications 5 à 18, où la composition est conditionnée dans un vaporisateur, un flacons pompe ou dans un récipient aérosol.

20. Procédé selon l'une quelconque des revendications 5 à 19, où le pH de la composition contenant le ou les corps gras insaturés de formule (I) varie de 1 à 11 et de préférence de 2 à 6.

21. Procédé selon l'une quelconque des revendications 5 à 20, comprenant une étape supplémentaire de rinçage et/ou une étape de lavage au shampoing avant ou après l'application de la composition contenant le ou les corps gras insaturés de formule (I).

22. Procédé selon l'une quelconque des revendications 5 à 21, comprenant une étape supplémentaire de séchage total ou partiel des fibres kératiniques avec un séchoir.

23. Procédé selon l'une quelconque des revendications 5 à 22 comprenant une étape supplémentaire de chauffage de la composition comprenant le ou les corps gras insaturé de formule (1) que l'on appliquera ensuite directement sur les fibres kératiniques.

24. Procédé selon l'une quelconque des revendications 5 à 22 comprenant une étape supplémentaire de chauffage des fibres kératiniques après application de la composition comprenant le ou les corps gras insaturé de formule (I).

25. Procédé selon la revendication 24, où le chauffage des fibres kératiniques est effectué au moyen d'un fer, d'un mélange eau liquide/vapeur d'eau ou bien au moyen d'un casque chauffant.

26. Procédé de coloration des fibres kératiniques, en particulier des fibres kératiniques humaines et plus particulièrement des cheveux, consistant à appliquer sur les fibres une composition (A) colorante directe ou d'oxydation pendant un temps suffisant pour développer la couleur, et de faire suivre ou précéder cette application par l'application d'une composition (B) contenant dans un milieu cosmétiquement acceptable au moins un corps gras insaturé de formule (I) telle que définie dans l'une quelconque des revendications précédentes.

27. Procédé selon la revendication 26, où l'application de la composition (A) est suivie d'un rinçage et/ou d'un séchage des fibres kératiniques.

28. Procédé selon la revendication 26 ou 27, où l'application de la composition (B) est d'un rinçage et/ou d'un séchage des fibres kératiniques et/ou d'un chauffage des fibres kératiniques.

29. Procédé selon la revendication 26 ou 27, où la composition (B) est préalablement chauffée.

30. Procédé selon l'une quelconque des revendications 26 à 29 où la composition (B) est appliquée après l'application de la composition (A) colorante directe ou d'oxydation, soit immédiatement, soit en différé, et les applications de ladite composition pouvant être répétées entre deux colorations.

31. Procédé selon l'une quelconque des revendications 26 à 30, où la composition colorante (A) résulte du mélange au moment de l'emploi d'une composition colorante (A₁) contenant au moins un colorant direct et d'une composition (A₂) contenant un agent oxydant.

32. Procédé selon l'une quelconque des revendications 26 à 31, où la composition colorante (A) résulte du mélange au moment de l'emploi d'une composition colorante (A₃) contenant au moins une base d'oxydation et éventuellement au moins un coupleur et/ou un colorant direct et d'une composition (A₄) contenant un agent oxydant.

33. Agent de coloration multi-composants comportant au moins un premier composant comprenant une composition (A) colorante directe ou d'oxydation et un deuxième composant comprenant une composition (B) contenant dans un milieu cosmétiquement acceptable au moins un corps gras insaturé de formule (I) telle que définie dans l'une quelconque des revendications précédentes.

34. Agent de coloration multi-composants comportant au moins un premier composant comprenant une composition (A₁) comprenant au moins un colorant direct, un deuxième composant comprenant une composition (A₂) contenant au moins un agent oxydant et un troisième composant comprenant une composition (B) contenant dans un milieu cosmétiquement acceptable au moins un corps gras insaturé de formule (I) telle que définie dans l'une quelconque des revendications précédentes.

35. Agent de coloration multi-composants comportant au moins un premier composant comprenant une composition (A₃) contenant au moins au moins une base d'oxydation et éventuellement au moins un coupleur et/ou au moins un colorant direct, un deuxième composant comprenant une composition (A₄) contenant au moins un agent oxydant et un troisième composant comprenant une composition (B) contenant dans un milieu cosmétiquement acceptable au moins un corps gras insaturé de formule (I) telle que définie dans l'une quelconque des revendications précédentes.

## Claims

1. Use of at least one unsaturated fatty substance of formula (I) below: 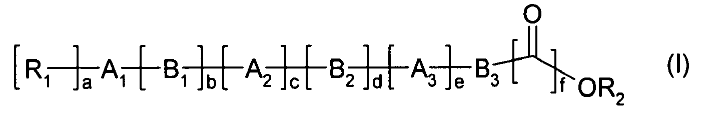 in which:
A₁, A₂ and A₃, which may be identical or different, denote a linear or cyclic, monovalent or divalent hydrocarbon-based radical comprising at least one unsaturation;
B₁, B₂ and B₃, which may be identical or different, denote a radical CₙH₂ₙ in which n is an integer less than 20;
R₁ denotes a linear or branched C₁-C₁₂ alkyl;
R₂ denotes hydrogen, an alkali metal M or a sorbitan group of formula: 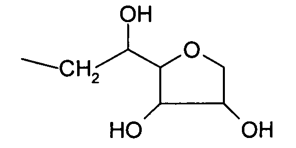
a, b, c, d, e and f, which may be identical or different, are equal to 0 or 1,
as an agent for protecting colour with respect to the washing of artificially dyed keratin fibres, especially human keratin fibres and more particularly the hair.

2. Use according to Claim 1, in which the compounds of formula (I) are chosen from the compounds of formula (II) below: 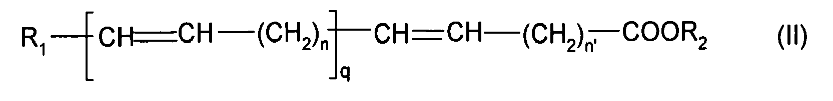 in which:
R₁ denotes a linear or branched C₁-C₁₂ alkyl;
R₂ denotes hydrogen, an alkali metal or a sorbitan group of formula: 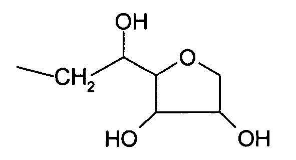
n and n', which may be identical or different, are integers ranging from 1 to 10 and preferably from 1 to 8;
q is equal to 0, 1 or 2.

3. Use according to Claim 1 or 2, in which the compound of formula (I) is chosen from:
lauroleic acid;
myristoleic acid;
palmitoleic acid;
oleic acid;
linoleic acid;
linolenic acid;
sorbitan oleate.

4. Use according to Claim 3, in which the compound of formula (I) is chosen from oleic acid, sorbitan oleate and linoleic acid.

5. Process for protecting colour with respect to the washing of artificially dyed keratin fibres, especially human keratin fibres and more particularly the hair, **characterized in that** it consists in applying to the said fibres, before or after dyeing, at least one composition comprising, in a cosmetically acceptable medium, at least one unsaturated fatty substance of formula (I) as defined according to any one of Claims 1 to 4.

6. Process according to Claim 5, in which the composition comprising, in a cosmetically acceptable medium, at least one unsaturated fatty substance of formula (I) is applied to the fibres after dyeing.

7. Process according to Claim 5 or 6, in which the cosmetically acceptable medium of the composition consists of water or of a mixture of water and of at least one cosmetically acceptable organic solvent.

8. Process according to Claim 7, in which the organic solvent is chosen from C₁-C₄ lower alkanols, such as ethanol and isopropanol; polyols and polyol ethers, and mixtures thereof.

9. Process according to Claim 7 or 8, in which the solvent(s) is (are) present in proportions ranging from 1% to 40% by weight approximately relative to the total weight of the composition, and even more preferentially from 3% to 30% by weight approximately relative to the total weight of the composition.

10. Process according to any one of Claims 5 to 9, in which the unsaturated fatty substance(s) of general formula (I) is (are) present in concentrations ranging from 1% to 100% by weight and more preferentially from 5% to 90% by weight relative to the total weight of the composition.

11. Process according to any one of Claims 5 to 10, in which the composition consists of 100% by weight of unsaturated fatty substance of formula (I).

12. Process according to any one of Claims 5 to 11, in which the composition contains at least one additive chosen from anionic, cationic, nonionic, amphoteric or zwitterionic surfactants, or mixtures thereof, anionic, cationic, nonionic, amphoteric or zwitterionic polymers, or mixtures thereof, mineral or organic thickeners, and in particular anionic, cationic, nonionic and amphoteric polymeric associative thickeners, penetrants, sequestrants, fragrances, buffers, dispersants, conditioning agents, for instance modified or unmodified, volatile or non-volatile silicones, film-forming agents, ceramides, preserving agents and opacifiers.

13. Process according to any one of Claims 5 to 12, in which the composition contains at least one agent for protecting against the effects of atmospheric agents, especially light.

14. Process according to Claim 13, in which the agent for protecting against the effects of atmospheric agents is chosen from organic UV-screening agents, free-radical scavengers and antioxidants.

15. Process according to any one of Claims 5 to 14, in which the composition contains at least one aromatic alcohol and at least one aromatic carboxylic acid.

16. Process according to any one of Claims 5 to 15, in which the composition contains at least one or more conditioning agents.

17. Process according to Claim 16, in which the conditioning agent(s) represent(s) from 0.001% to 20% by weight, preferably from 0.01% to 10% by weight and more particularly from 0.1% to 3% by weight relative to the total weight of the composition.

18. Process according to any one of Claims 5 to 17, in which the composition is in the form of an aqueous or aqueous-alcoholic lotion or in the form of an oil, a gel, a milk, a cream, an emulsion or a mousse.

19. Process according to any one of Claims 5 to 18, in which the composition is conditioned in a vaporizer, in a pump-dispenser bottle or in an aerosol container.

20. Process according to any one of Claims 5 to 19, in which the pH of the composition containing the unsaturated fatty substance(s) of formula (I) ranges from 1 to 11 and preferably from 2 to 6.

21. Process according to any one of Claims 5 to 20, comprising an additional rinsing step and/or a step of washing with shampoo before or after applying the composition containing the unsaturated fatty substance(s) of formula (I).

22. Process according to any one of Claims 5 to 21, comprising an additional step of total or partial drying of the keratin fibres with a hairdryer.

23. Process according to any one of Claims 5 to 22, comprising an additional step of heating the composition comprising the unsaturated fatty substance(s) of formula (I), which will then be applied directly to the keratin fibres.

24. Process according to any one of Claims 5 to 22, comprising an additional step of heating the keratin fibres after applying the composition comprising the unsaturated fatty substance(s) of formula (I).

25. Process according to Claim 24, in which the heating of the keratin fibres is performed using an iron, a liquid water/steam mixture or using a heating hood.

26. Process for dyeing keratin fibres, in particular human keratin fibres and more particularly the hair, which consists in applying to the fibres a direct or oxidation dye composition (A) for a time that is sufficient to develop the colour, and in following or preceding this application by the application of a composition (B) containing, in a cosmetically acceptable medium, at least one unsaturated fatty substance of formula (I) as defined in any one of the preceding claims.

27. Process according to Claim 26, in which the application of composition (A) is followed by rinsing and/or drying of the keratin fibres.

28. Process according to Claim 26 or 27, in which the application of composition (B) is followed by rinsing and/or drying of the keratin fibres and/or by heating of the keratin fibres.

29. Process according to Claim 26 or 27, in which composition (B) is preheated.

30. Process according to any one of Claims 26 to 29, in which composition (B) is applied after applying the direct or oxidation dye composition (A), either immediately or after a delay, and the applications of the said composition possibly being repeated between two colorations.

31. Process according to any one of Claims 26 to 30, in which the dye composition (A) results from the mixing, at the time of use, of a dye composition (A₁) containing at least one direct dye, and of a composition (A₂) containing an oxidizing agent.

32. Process according to any one of Claims 26 to 31, in which the dye composition (A) results from the mixing, at the time of use, of a dye composition (A₃) containing at least one oxidation base and optionally at least one coupler and/or one direct dye, and of a composition (A₄) containing an oxidizing agent.

33. Multi-component dyeing agent comprising at least a first component comprising a direct or oxidation dye composition (A) and a second component comprising a composition (B) containing, in a cosmetically acceptable medium, at least one unsaturated fatty substance of formula (I) as defined in any one of the preceding claims.

34. Multi-component dyeing agent comprising at least a first component comprising a composition (A₁) comprising at least one direct dye, a second component comprising a composition (A₂) containing at least one oxidizing agent, and a third component comprising a composition (B) containing, in a cosmetically acceptable medium, at least one unsaturated fatty substance of formula (I) as defined in any one of the preceding claims.

35. Multi-component dyeing agent comprising at least a first component comprising a composition (A₃) containing at least one oxidation base and optionally at least one coupler and/or at least one direct dye, a second component comprising a composition (A₄) containing at least one oxidizing agent, and a third component comprising a composition (B) containing, in a cosmetically acceptable medium, at least one unsaturated fatty substance of formula (I) as defined in any one of the preceding claims.

## Patentansprüche

1. Verwendung mindestens eines ungesättigten Fettstoffs der nachstehenden Formel (I) 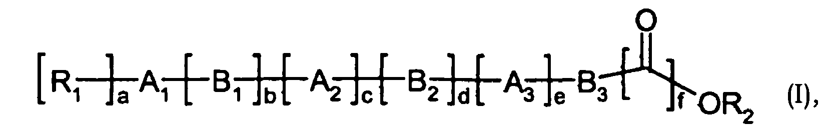 worin bedeuten:
A₁, A₂ und A₃, die gleich oder verschieden sind, eine einwertige oder zweiwertige geradkettige oder cyclische Kohlenwasserstoffgruppe, die mindestens eine ungesättigte Bindung enthält,
B₁, B₂ und B₃, die gleich oder verschieden sind, eine Gruppe CₙH₂ₙ, in der n eine ganze Zahl von weniger als 20 bedeutet,
R₁ geradkettiges oder verzweigtes C₁-C₁₂-Alkyl, R₂ Wasserstoff, ein Alkalimetall M oder die Sorbitangruppe der Formel 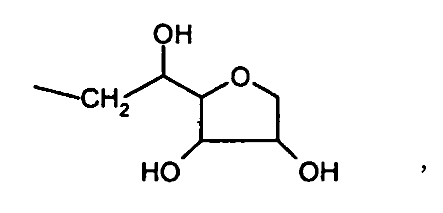 und
a, b, c, d, e und f, die gleich oder verschieden sind, 0 oder 1,
als Mittel, das einen Schutz der Farbe von künstlich gefärbten Keratinfasern, insbesondere menschlichen Keratinfasern und ganz besonders der Haare, gegen Auswaschen erlaubt.

2. Verwendung nach Anspruch 1, wobei die Verbindungen der Formel (I) unter Verbindungen der nachstehenden Formel (II) ausgewählt sind, 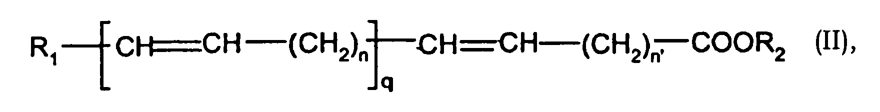 worin bedeuten:
R₁ geradkettiges oder verzweigtes C₁-C₁₂-Alkyl,
R₂ Wasserstoff, ein Alkalimetall oder die Sorbitangruppe der Formel 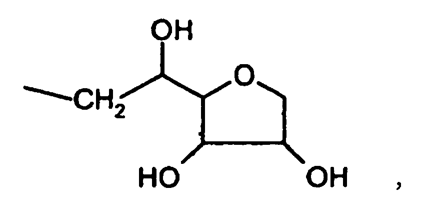
n und n', die gleich oder verschieden sind, ganze Zahlen von 1 bis 10 und vorzugsweise von 1 bis 8
und
q 0, 1 oder 2.

3. Verwendung nach Anspruch 1 oder 2, wobei die Verbindung der Formel (I) ausgewählt ist unter
Lauroleinsäure,
Myristoleinsäure,
Palmitoleinsäure,
Ölsäure,
Linolsäure,
Linolensäure und
Sorbitanoleat.

4. Verwendung nach Anspruch 3, wobei die Verbindung der Formel (I) unter Ölsäure, Sorbitanoleat und Linolsäure ausgewählt ist.

5. Verfahren zum Schutz der Farbe von künstlich gefärbten Keratinfasern, insbesondere menschlichen Keratinfasern und ganz besonders der Haare, gegen Auswaschen, **dadurch gekennzeichnet, dass** es darin besteht, mindestens eine Zusammensetzung, die in einem kosmetisch akzeptablen Medium mindestens einen ungesättigten Fettstoff der Formel (I), wie er in einem der Ansprüche 1 bis 4 definiert ist, enthält, vor oder nach dem Färben auf diese Fasern aufzubringen.

6. Verfahren nach Anspruch 5, bei dem die Zusammensetzung, die in einem kosmetisch akzeptablen Medium mindestens einen ungesättigten Fettstoff der Formel (I) enthält, nach dem Färben auf die Fasern aufgebracht wird.

7. Verfahren nach Anspruch 5 oder 6, bei dem das kosmetisch akzeptable Medium der Zusammensetzung aus Wasser oder einem Gemisch von Wasser mit mindestens einem kosmetisch akzeptablen organischen Lösungsmittel besteht.

8. Verfahren nach Anspruch 7, bei dem das organische Lösungsmittel ausgewählt wird unter niederen C₁-C₄-Alkanolen, wie Ethanol und Isopropanol, Polyolen und Polyolethern sowie Gemischen dieser Verbindungen.

9. Verfahren nach Anspruch 7 oder 8, bei dem das Lösungsmittel oder die Lösungsmittel in Mengenanteilen von etwa 1 bis 40 Gew.-%, bezogen auf das Gesamtgewicht der Zusammensetzung, und noch bevorzugter von etwa 3 bis 30 Gew.-% vorliegen, bezogen auf das Gesamtgewicht der Zusammensetzung.

10. Verfahren nach einem der Ansprüche 5 bis 9, bei dem der ungesättigte Fettstoff oder die ungesättigten Fettstoffe der allgemeinen Formel (I) in Konzentrationen von 1 bis 100 Gew.-% und noch bevorzugter von 5 bis 90 Gew.-% vorliegen, bezogen auf das Gesamtgewicht der Zusammensetzung.

11. Verfahren nach einem der Ansprüche 5 bis 10, bei dem die Zusammensetzung zu 100 Gew.-% aus dem ungesättigten Fettstoff der Formel (I) besteht.

12. Verfahren nach einem der Ansprüche 5 bis 11, wobei die Zusammensetzung mindestens einen Zusatzstoff enthält, der ausgewählt ist unter anionischen, kationischen, nichtionischen, amphoteren und zwitterionischen Tensiden oder ihren Gemischen, anionischen, kationischen, nichtionischen, amphoteren und zwitterionischen Polymeren oder ihren Gemischen, anorganischen oder organischen Verdickungsmitteln und insbesondere polymeren, anionischen, kationischen, nichtionischen und amphoteren assoziativen Verdickungsmitteln, Penetrationsmitteln, Sequestrierungsmitteln, Parfums, Puffern, Dispergiermitteln, Konditioniermitteln, wie beispielsweise flüchtigen oder nichtflüchtigen, modifizierten oder unmodifizierten Siliconen, filmbildenden Mitteln, Ceramiden, Konservierungsmitteln und Trübungsmitteln.

13. Verfahren nach einem der Ansprüche 5 bis 12, bei dem die Zusammensetzung mindestens ein Schutzmittel gegen atmosphärische Einflüsse wie Licht enthält.

14. Verfahren nach Anspruch 13, bei dem das Schutzmittel gegen atmosphärische Einflüsse ausgewählt ist unter organischen UV-Filtern, Mitteln gegen Radikale und Antioxidationsmitteln.

15. Verfahren nach einem der Ansprüche 5 bis 14, wobei die Zusammensetzung mindestens einen aromatischen Alkohol und mindestens eine aromatische Carbonsäure enthält.

16. Verfahren nach einem der Ansprüche 5 bis 15, bei dem die Zusammensetzung mindestens ein Konditionierungsmittel oder mehrere Konditionierungsmittel enthält.

17. Verfahren nach Anspruch 16, wobei das oder die Konditionierungsmittel 0,001 bis 20 Gew.-%, vorzugsweise 0,01 bis 10 Gew.-% und noch bevorzugter 0,1 bis 3 Gew.-% ausmachen, bezogen auf das Gesamtgewicht der Zusammen - setzung.

18. Verfahren nach einem der Ansprüche 5 bis 17, bei dem die Zusammensetzung in Form einer wässerigen oder wässerig - alkoholischen Lotion, in Form eines Öls, eines Gels, einer Milch, einer Creme, einer Emulsion oder eines Schaums vorliegt.

19. Verfahren nach einem der Ansprüche 5 bis 18, bei dem die Zusammensetzung in einem Spray, einem Pumpflakon oder in einem Aerosolbehälter konfektioniert ist.

20. Verfahren nach einem der Ansprüche 5 bis 19, wobei der pH-Wert der Zusammensetzung, die den ungesättigten Fettstoff ode r die ungesättigten Fettstoffe der Formel (I) enthält, im Bereich von 1 bis 11 und vorzugsweise im Bereich von 2 bis 6 liegt.

21. Verfahren nach einem der Ansprüche 5 bis 20, das eine zusätzliche Stufe des Spülens und/oder eine Stufe des Waschens beim Shampoonieren vor oder nach Anwendung der Zusammensetzung, die den ungesättigten Fettstoff oder die ungesättigten Fettstoffe der Formel (I) enthält, umfasst.

22. Verfahren nach einem der Ansprüche 5 bis 21, das eine zusätzliche Stufe des vollständigen oder teilweisen Trocknens der Keratinfasern mit einem Fön umfasst.

23. Verfahren nach einem der Ansprüche 5 bis 22, das eine zusätzliche Stufe des Erwärmens der Zusammensetzung, die den ungesättigten Fettstoff oder die ungesättigten Fettstoffe der Formel (I) enthält, umfasst, die anschließend direkt auf die Keratinfasern aufgebracht wird.

24. Verfahren nach einem der Ansprüche 5 bis 22, das eine zusätzliche Stufe des Erwärmens der Keratinfasern nach dem Aufbringen der Zusammensetzung umfasst, die den ungesättigten Fettstoff oder die ungesättigten Fettstoff e der Formel (I) enthält.

25. Verfahren nach Anspruch 24, bei dem das Erwärmen der Keratinfasern mit einem Frisierstab, einem Gemisch von flüssigem und dampfförmigem Wasser oder mit einer Trockenhaube vorgenommen wird.

26. Verfahren zum Färben von Keratinfasern, insbesondere menschlichen Keratinfasern und ganz besonders der Haare, das darin besteht, eine direktziehende oder auf Oxidationsfä rbung beruhende Färbezusammensetzung (A) während einer zur Entwicklung der Färbung ausreichenden Zeit auf die Fasern aufzubringen und vor oder nach diesem Aufbringen eine Zusammensetzung (B) aufzubringen, die in einem kosmetisch akzeptablen Medium mindestens einen ungesättigten Fettstoff der Formel (I) enthält, wie in einem der vorhergehenden Ansprüche definiert ist.

27. Verfahren nach Anspruch 26, bei dem sich an das Aufbringen der Zusammensetzung (A) ein Spülen und/oder Trocknen der Keratinfasern anschließt.

28. Verfahren nach Anspruch 26 oder 27, bei dem nach dem Aufbringen der Zusammensetzung (B) die Keratinfasern gespült und/oder getrocknet und/oder erwärmt werden.

29. Verfahren nach Anspruch 26 oder 27, bei dem die Zusammensetzung (B) vorher erwärmt wird.

30. Verfahren nach einem der Ansprüche 26 bis 29, bei dem die Zusammensetzung (B) nach dem Aufbringen der direktziehenden oder auf Oxidationsfärbung beruhenden Zusammensetzung (A) entweder unmittelbar oder in einem Zeitabstand aufgebracht wird und die Anwendung dieser Zusammensetzung zwischen zwei Färbungen wiederholt werden kann.

31. Verfahren nach einem der Ansprüche 26 bis 30, bei dem die Färbezusammensetzung (A) aus dem Mischen einer Färbezusammensetzung (A₁), die mindestens einen direktziehenden Farbstoff enthält, und einer Zusammensetzung (A₂), die ein Oxidationsmittel enthält, bei der Anwendung resultiert.

32. Verfahren nach einem der Ansprüche 26 bis 31, bei dem die Färbezusammensetzung (A) aus dem Mischen einer Färbe - zusammensetzung (A₃), die mindestens eine Oxidationsbase und gegebenenfalls mindestens einen Kuppler und/oder einen direktziehenden Farbstoff enthält, und einer Zusammensetzung (A₄), die ein Oxidationsmittel enthält, bei der Anwendung resultiert.

33. Mehrkomponenten-Färbemittel, das aufweist: mindestens einen ersten Bestandteil, der eine auf Direktfärbung oder Oxidationsfärbung beruhende Zusammensetzung (A) enthält, und einen zweiten Bestandteil, der eine Zusammensetzung (B) enthält, die in einem kosmetisch akzeptablen Medium mindestens einen ungesättigten Fettstoff der Formel (I), wie in einem der vorher - gehenden Ansprüche definiert, enthält.

34. Mehrkomponenten-Färbemittel, das aufweist: mindestens einen ersten Bestandteil, der eine Zusammensetzung (A₁) aufweist, die mindestens einen direktziehenden Farbstoff enthält, einen zweiten Bestandteil, der eine Zusammensetzung (A₂) aufweist, die mindestens ein Oxidationsmittel enthält, und einen dritten Bestandteil, der eine Zusammensetzung (B) aufweist, die in einem kosmetisch akzeptablen Medium mindestens einen ungesättigten Fettstoff der Formel (I), wie in einem der vorhergehenden Ansprüche definiert, enthält.

35. Mehrkomponenten-Färbemittel, das aufweist: mindestens einen ersten Bestandteil, der eine Zusammensetzung (A₃) aufweist, die mindestens eine Oxidationsbase und gegebenenfalls mindestens einen Kuppler und/oder mindestens einen direktziehenden Farbstoff enthält, einen zweiten Bestandteil, der eine Zusammensetzung (A₄) aufweist, die mindestens ein Oxidationsmittel enthält, und einen dritten Bestandteil, der eine Zusammensetzung (B) aufweist, die in einem kosmetisch akzeptablen Medium mindestens einen ungesättigten Fettstoff der Formel (I), wie in einem der vorhergehenden Ansprüche definiert, enthält.
